# EUROPÄISCHE PATENTSCHRIFT

(11) **EP 4 547 129 B1**
(45) Veröffentlichungstag und Bekanntmachung des Hinweises auf die Patenterteilung: **06.05.2026**
(21) Anmeldenummer: 23737929.2
(22) Anmeldetag: 29.06.2023
(51) Int. Cl.: A61B 17/29

(54) **CHIRURGISCHES INSTRUMENT UND VERFAHREN ZU DESSEN ZERLEGUNG**
SURGICAL INSTRUMENT AND METHOD FOR TAKING SAME APART
INSTRUMENT CHIRURGICAL ET PROCÉDÉ POUR LE DÉMONTER

(30) Priorität: 30.06.2022 DE 102022116372
(43) Veröffentlichungstag der Anmeldung: 07.05.2025
(73) Patentinhaber: Karl Storz SE & Co. KG, 78532 Tuttlingen (DE)
(72) Erfinder: PÖNISCH, Judith, 78532 Tuttlingen (DE); KÄRCHER, Daniel, 78532 Tuttlingen (DE); LÄNGLE, Dominik, 78532 Tuttlingen (DE); MERZ, Robin, 78532 Tuttlingen (DE); SCHNEIDER, Janosz, 78532 Tuttlingen (DE); SCHNEIDER, Sven, 78532 Tuttlingen (DE); UNGER, Tobias, 78532 Tuttlingen (DE)
(86) Internationale Anmeldenummer: PCT/EP2023/067770
(87) Internationale Veröffentlichungsnummer: WO 2024/003216

(56) Entgegenhaltungen:
- EP-B1- 2 777 537
- DE-A1- 102013 104 789
- DE-C1- 19 707 373
- US-B2- 9 724 161

## Beschreibung

Die Erfindung betrifft ein chirurgisches Instrument und ein Verfahren, um dieses zu zerlegen.

Aus dem Stand der Technik sind vielfältige chirurgische Instrumente bekannt, die es vorsehen, ein chirurgisches Werkzeug wie ein Endoskop, eine Zange oder ähnliches an einen Schaft anzudocken. Nach der Operation oder Behandlung des Patienten muss das chirurgische Instrument fachgerecht gereinigt werden und hierfür müssen Werkzeug und Schaft voneinander gelöst werden. Diese Zerlegung ist vielfach aufwändig.

Ein solches chirurgisches Instrument ist etwa aus DE 197 07 373 C1 bekannt; dort stellt eine Bajonettkupplung eine lösbare Verbindung zweiter Rohrschaftinstrumente oder Instrumententeile bereit.

Ausgehend von diesem Stand der Technik ist es Aufgabe der vorliegenden Erfindung, ein chirurgisches Instrument bereitzustellen, dass bezüglich des Trennens und Verbindens von Werkzeug und Schaft verbessert ist, weil nutzerfreundlicher und einfacher.

Diese Aufgabe wird durch ein chirurgisches Instrument mit den Merkmalen des Anspruchs 1 gelöst.

Bevorzugte Ausführungsformen des chirurgischen Instruments sind in den Unteransprüchen ausgeführt.

Die weitere Aufgabe betrifft die Schaffung eines verbesserten Zerlegeverfahrens solcher chirurgischen Instrumente.

Diese Aufgabe wird durch das Verfahren mit den Merkmalen des unabhängigen Anspruchs 6 gelöst.

Gemäß einer ersten Ausführungsform des erfindungsgemäßen chirurgischen Instruments weist dieses einen Schaft auf, an dessen distalem Ende ein Werkzeug lösbar gehalten ist. In dem Schaft ist eine Zugstange längs bewegbar zum Bewegen des Werkzeugs angeordnet.

Die Zugstange ist von einem Gabelelement distalseitig und von dem Schaft proximalseitig umgeben. Das Gabelelement weist an seiner dem Schaft zugewandten Seite einen Endabschnitt auf. Benachbart zu bzw. nahe bei dem proximalen Ende des Gabelelements hat dieses zumindest einen Hinterschnitt. Der Schaft weist an seiner dem Gabelelement zugewandten Seite eine Schulter auf, von der aus sich ein Endabschnitt mit verringertem Durchmesser erstreckt, wobei entlang des Endabschnitts sich zumindest eine Ausnehmung erstreckt. Der Innendurchmesser des Schafts ist entlang einem distalen Teil des Endabschnitts aufgeweitet. In die zumindest eine Ausnehmung des Schafts ist ein längliches Federplättchen eingelegt, das an seinem zu dem Gabelelement weisenden Ende eine radial nach innen weisende Rastnase aufweist. Die Rastnase steht jeweils mit dem zumindest einen Hinterschnitt des Gabelelements in Eingriff. Die Zugstange hat an ihrem vor dem proximalen Ende des Gabelelements liegenden Abschnitt eine umfängliche Verdickung ihres Durchmessers, und das Federplättchen hat einen Quersteg, der zu der Zugstange weist und zwischen dem Ende des Gabelelements und der umfänglichen Verdickung der Zugstange liegt.

"Distalseitig" meint hierin immer bedienerfern, wobei es gleich ist, ob der Bediener ein Mensch oder eine Maschine, also ein Roboter, ist. Für "proximalseitig" gilt folglich das Umgekehrte.

Durch die erfindungsgemäße Vorrichtung wird vorteilhaft eine Rastverbindung bereitgestellt, wobei die Rastnase, die durch das Federplättchen federnd ausgebildet ist, in einer Montageanordnung des chirurgischen Instruments in den Hinterschnitt des Gabelelements eingreift.

Gabelelement und Schaft sind gegeneinander gut geführt und gegen Rotation konstruktiv gesperrt, so dass eine einfache Zerlegung des Schaftes vom Werkzeug erreicht wird, indem am proximalen Ende Druck auf die Zugstange ausgeübt und diese in distale Richtung verschoben wird. Die Durchmesserverdickung der Zugstange wirkt auf den Quersteg des Federplättchens und drückt die Rastnase radial nach außen.

Die Zugstange erreicht eine Überoffenposition, was aus dem vergrößerten Öffnungswinkel bspw. der Maulteile des Werkzeugs ersichtlich ist, und bewegt die federnde Rastnase nach außen aus dem Hinterschnitt des Gabelelements heraus. Mit dieser Bewegung wird auch das Gabelelement aus dem Schaft bewegt. Eine Verdrehbewegung, wie es bei den aus dem Stand der Technik bekannten Bajonettverbindungen üblich ist, ist vorteilhaft nicht notwendig, so dass die Demontage sehr einfach ist; das gilt ebenfalls für die Montage: Das Werkzeug, beispielsweise ein Zangeneinsatz, wird mit seinem distalen Ende in einer Nichtbenutzungsanordnung (bei einem Zangeneinsatz wären das etwa geschlossene Zangen) von distalseitig einfach axial in den Schaft geschoben und von der Rastnase des Federplättchens gefasst. Die federnde Rastnase springt quasi durch ihre Vorspannung in den Hinterschnitt an dem Gabelelement. Die Verbindung ist so von Beginn an direkt gesichert.

Wenn gelöst wird, wird auf einfache Weise das Gegenteil erreicht, indem die vorstehend erwähnte Überoffenstellung hergestellt wird, die die Rastnase wieder aus dem Eingriff mit den Hinterschnitt nimmt. Dies wird erreicht, indem durch den proximalseitigen Druck auf die Zugstange eine axiale Kraft wirkt und die Durchmesserverdickung der Zugstange auf den Quersteg des Federplättchens wirkt und die Rastnase radial nach außen drückt.

In einer bevorzugten Ausführungsform des erfindungsgemäßen chirurgischen Elements weist das Gabelelement eine Schulter auf, von der aus sich der Endabschnitt mit einem verringerten Durchmesser erstreckt - so kann ein Gabelelement realisiert werden, dessen anbringbares Werkzeugteil den gleichen Außendurchmesser wie das Gabelelement hat.

In einer weiteren bevorzugten Ausführungsform des erfindungsgemäßen chirurgischen Elements weist der Endabschnitt des Gabelelements zwei Hinterschnitte auf, die an gegenüberliegenden Seiten des zylindrischen Endabschnitts angeordnet sind. Korrespondierend dazu weist der Endabschnitt des Schafts zwei Ausnehmungen auf, in denen jeweils ein Federplättchen liegt, so dass in dieser Variante zwei Federplättchen vorliegen, deren Rastnasen in die beiden Hinterschnitte des Gabelelements eingreifen. Die beiden Kombinationen zwischen Ausnehmung, Federplättchen und Hinterschnitt sind in einer Montageanordnung an voneinander abgewandten Seiten der Zugstange angeordnet, womit eine stabile Bauteilverbindung erreicht werden kann.

Die zumindest eine Ausnehmung des Schafts kann nach Art eines Langlochs gestaltet sein. Dabei kann die Ausnehmung zu einem Ende offen oder geschlossen (quasi eine Ausnehmung mit geschlossener Umrandung) gestaltet sein. Sie bildet die Form des oder der Federplättchen nach und sorgt für eine Führung für die Bewegung der Rastnase radial nach außen bei Zerlegung des Schafts von dem Werkzeug.

In noch einer weiteren Ausführungsform des erfindungsgemäßen chirurgischen Instruments weist die Ausnehmung des Schafts proximalseitig einen Abschnitt auf, in dem eine Auflagefläche für ein proximales Ende des einzulegenden Federplättchens ausgebildet ist. Das Federplättchen wird auf die Auflagefläche einfach eingelegt bzw. kann eingeklebt, verschraubt, verschweißt oder anderweitig befestigt sein.

Die umfängliche Verdickung der Zugstange kann ringförmig, kugelig oder kegelig sein. Dabei ist es möglich, dass die Verdickung der Zugstange auch nur teilumfänglich in den Abschnitten, die an das oder die Federplättchen benachbart sind, vorliegt. Der Quersteg des Federplättchens weist eine zu der umfänglichen Verdickung korrespondierende Form auf, so dass die umfängliche Verdickung der Zugstange direkt mit dem Quersteg des Federplättchens zusammenwirken kann, wenn die Zugstange zur Zerlegung des chirurgischen Instruments distal verschoben wird. Der Quersteg des Federplättchens kann ein aufgesetzter Steg, eine zu dem Gabelelement gerichtete Materialverdickung, eine Neigung des Federplättchens oder eine darin eingebrachte Biegung sein.

Üblicherweise wird eine Werkzeugmechanik einenends distalseitig mit dem Werkzeug und andernends proximalseitig mit der Zugstange in Wirkverbindung stehen, um das Werkzeug über die Zugstange bewegen zu können. Über das Gabelelement ist das Werkzeug mit dem Schaft verbunden, sodass die Zugstange über die Werkzeugmechanik und das Werkzeug mit dem Gabelelement verbunden ist. Das Werkzeug, also bspw. die Maulteile ist bzw. sind im Gabelelement gelagert, das fest mit dem Schaft verbunden ist. Die Zugstange erstreckt sich axial bewegbar und rotierbar durch den Schaft in das Gabelelement zu der Mechanik, die mit den Maulteilen verbunden ist.

Nach einer weiteren Ausführungsform des erfindungsgemäßen chirurgischen Instruments können sich entlang dem Endabschnitt des Gabelelements zwei Führungsschienenelemente erstrecken, während benachbart zu dem Ende des Gabelelements der zumindest eine bzw. die Hinterschnitt(e) vorliegen. Entlang dem Endabschnitt des Schafts erstrecken sich zwei Führungsschienenelemente von dessen distalen Ende in Richtung der Schulter. Die beiden Führungsschienenelemente des Schafts wirken verschiebbar jeweils mit den Führungsschienenelementen des Gabelelements zusammen und unterstützen so vorteilhaft die Verdrehsicherheit von Gabelelement und Schaft.

Nach noch einer weiteren Ausführungsform des erfindungsgemäßen chirurgischen Instruments sind die Führungsschienenelemente des Schafts Ausnehmungen oder Stege, und sind so ausgebildet, dass sie mit den Führungsschienenelementen des Gabelelements, die Stege oder Ausnehmungen oder Rillen sind, korrespondieren und zusammenwirken. Diese Art der Führungsschienenelemente bieten eine gute lineare Führung der Bauteile Gabelelement und Endabschnitt des Schafts, sowohl bei Zerlegung als auch bei Montage des chirurgischen Instruments.

Schließlich sind nach noch einer weiteren Ausführungsform des erfindungsgemäßen chirurgischen Instruments die Führungsschienenelemente des Schafts zu den Ausnehmungen des Schafts um 90 Grad zueinander versetzt angeordnet. Diese Anordnung bietet eine gute Stabilität bei Zerlegung bzw. Montage.

Nach einer weiteren Ausführungsform des erfindungsgemäßen chirurgischen Instruments ist in einem Montagezustand ein Überwurfrohr um den Endabschnitt des Gabelelements und um den Endabschnitt des Schafts angeordnet. Das Überwurfrohr weist ferner zumindest eine, bevorzugt zwei geschlossene Ausnehmung(en) auf, die über der oder den Ausnehmung(en) des Endabschnitts des Schafts liegen, wenn alle Bauteile in einer Montageanordnung vorliegen, d. h. die Rastnase des jeweiligen Federplättchens in den jeweiligen Hinterschnitt eingreift. Die geschlossene Ausnehmung des Überwurfrohrs überlappt sich nur in einem Teilbereich mit der des Endabschnitts des Schafts und endet vor der Auflagefläche für die proximalen Enden des jeweiligen Federplättchen. Dadurch dass das Überwurfrohr diesen Abschnitt überdeckt, wird das Federplättchen vor Herausfallen gesichert. Die geschlossene Ausnehmung des Überwurfrohrs bildet eine zusätzliche Führung für die lineare Bewegung des Federplättchens.

Ein erfindungsgemäßes Verfahren zur Zerlegung eines erfindungsgemäßen chirurgisches Instruments umfasst die Schritte
- in axialer Richtung am proximalen Ende der Zugstange Druck ausüben auf die Zugstange und dabei bewegen der Zugstange in distaler Richtung,
- dadurch bewegen der umfänglichen Verdickung der Zugstange gegen den Quersteg des zumindest einen Federplättchen und bis in eine Überoffenposition der Zugstange, dabei
- radial nach außen bewegen der Rastnase aus dem zumindest einen Hinterschnitt des Gabelelements und
- durch das Lösen der Verbindung von Gabelelement und Schaft lösen des Werkzeugs.

Da das Gabelelement bei Montage des chirurgischen Instruments nur linear in den Endabschnitt des Schafts eingeführt wurde, sorgt die lineare Bewegung der Zugstange in distaler Richtung gleichzeitig für eine komplette und damit einfache Zerlegung der Komponenten. Für die Montage gilt das Umgekehrte, wie bereits beschrieben. In einer alternativen Ausführungsform des Verfahrens kann an der Zugstange auch Zug ausgeübt werden, um die Zerlegung der Komponenten zu bewirken.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die in der Beschreibung und den Zeichnungen zu entnehmende Merkmale können einzeln für sich oder zu mehreren in beliebiger Kombination erfindungsgemäß angewandt werden, ohne den Rahmen der vorliegenden Offenbarung zu verlassen. Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Dabei zeigen:
- Fig. 1: eine schematische Ansicht des erfindungsgemäßen chirurgischen Instruments,
- Fig. 2: eine Schnittansicht des Schafts mit Zugstange und montiertem Gabelelement, und
- Fig. 3: eine perspektivische Explosionsansicht der Bauteile der Verbindung zwischen Gabelelement und Schaft des chirurgischen Instruments mit innenliegender Zugstange.

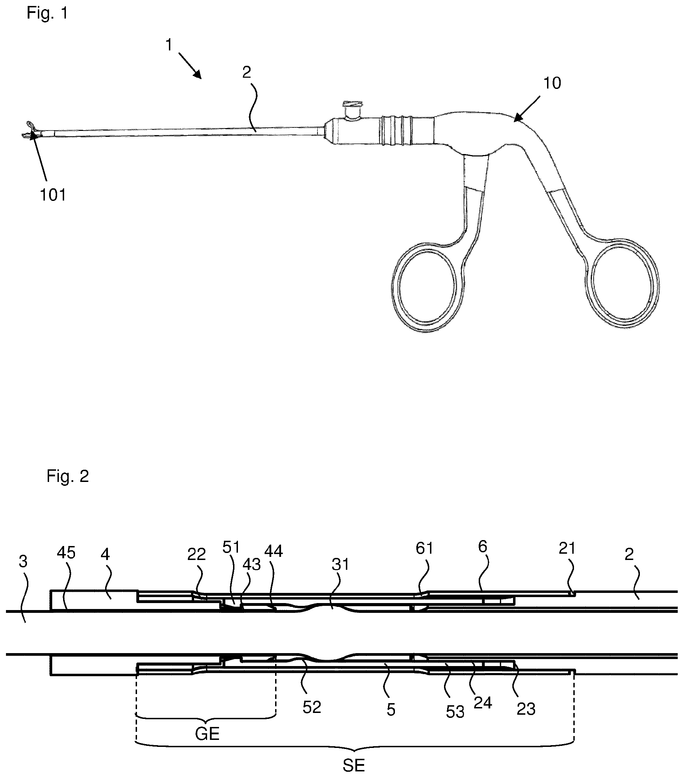

In Fig. 1 weist ein chirurgisches Instrument 1 mit einen Schaft 2 auf, der proximalseitig an einem Griff 10 angeordnet ist. Der Griff 10 ist in dieser Figur beispielhaft gezeigt, es kann auch eine anders gestaltete Handhabe oder bspw. ein Roboterarm eingesetzt werden. Distalseitig ist der Schaft 2 mit einem Werkzeug 101 verbunden, das ausgetauscht werden kann. Es ist dazu lösbar gehalten und das chirurgische Instrument 1 kann auf einfache Weise zerlegt werden.

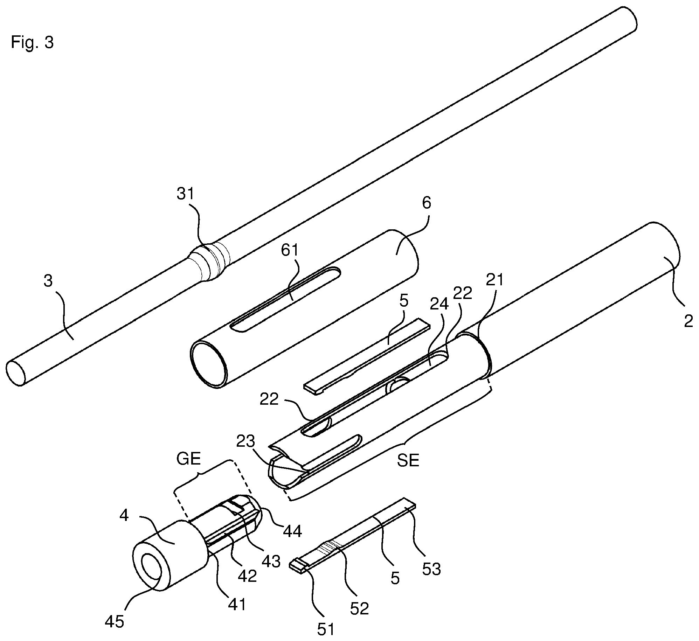

Wie dazu in Fig. 2 und 3 gezeigt ist, ist innerhalb des Schafts 2 eine längsbewegbare Zugstange 3 zum Bewegen des Werkzeugs 101 angeordnet. An ihrem proximalen Ende kann die Zugstange 3 mit dem Griff 10 verbunden werden (figurativ nicht dargestellt).

Die Zugstange 3 ist in einem distalen Endabschnitt von einem Gabelelement 4 umgeben, beide sind zylindrisch ausgebildet. Das Gabelelement 4 weist eine innere Bohrung 45 auf, deren Innendurchmesser dem Außendurchmesser der Zugstange 3 entspricht, so dass die Zugstange 3 durch das Gabelelement 4 hindurchgeführt ist. "Entsprechend" heißt hierin, dass die innere Bohrung 45 etwas größer bemessen ist als der Außendurchmesser der Zugstange 3, so dass die Zugstange 3 linear bewegt werden kann. Das Gabelelement 4 weist an seiner dem Schaft 2 zugewandten Seite eine Schulter 41 auf, von der aus sich ein Endabschnitt GE erstreckt (in proximaler Richtung in Fig. 2). Der Endabschnitt GE hat dabei einen verringerten Außendurchmesser und weist benachbart zu seinem Ende 44 zwei Hinterschnitte 43 auf, in denen der Außendurchmesser nochmals verringert ist. Das Ende 44 des Endabschnitts GE ist hierbei wieder verdickt, so dass zwei im Längsschnitt der Fig. 2 ersichtliche rechteckige Ausnehmungen, die Hinterschnitte 43, gebildet sind. Das Ende 44 des Endabschnitts GE ist angeschrägt, um eine Führung des Gabelelements 4 in den Schaft 2 zu erleichtern. Der Schaft 2 dagegen hat an seiner dem Gabelelement 4 zugewandten Seite eine erste Schulter 21. Von dieser Schulter 21 erstreckt sich in distaler Richtung ein Endabschnitt SE des Schafts 2 mit verringertem Durchmesser. Entlang des Endabschnitts SE des Schafts 2 erstrecken sich zwei geschlossene Ausnehmungen 22, die langlochartig ausgebildet sind, wie insbesondere in Fig. 3 gezeigt ist. Der Schaft 2 weist eine zweite Schulter 23 auf, von der aus sich der Außendurchmesser des Schafts 2 verringert und eine Auflagefläche 24 gebildet wird. In jede Ausnehmung 22 des Schafts 2 ist ein längliches Federplättchen 5 eingelegt, wobei ein proximales Ende 53 des Federplättchens 5 auf der Auflagefläche 24 aufliegt. Jedes Federplättchen 5 weist an seinem zu dem Gabelelement 4 weisenden Ende eine radial nach innen weisende Rastnase 51 auf, die in Fig. 2 jeweils mit einem der Hinterschnitte 43 des Gabelelements 4 in Eingriff steht.

Die Zugstange 3 hat an ihrem vor dem proximalen Ende des Gabelelements 4 liegenden Abschnitts eine umfängliche, kugelige Verdickung 31 ihres Durchmessers. Hierzu korrespondierend weist jedes Federplättchen 5 einen Quersteg 52 auf, der, wie in Fig. 2 gezeigt ist, radial nach innen zu der Zugstange 3 weist und zwischen dem Ende des Gabelelements 4 und der ringförmigen Verdickung 31 der Zugstange 3 liegt.

Damit die Zugstange 3 für eine Montage des chirurgischen Instruments 1 von distaler Seite in den Schaft 2 eingeschoben werden kann, ist ein Innendurchmesser des Schafts 2 entlang einem distalen Teil des Endabschnitts SE aufgeweitet. Er hat dabei einen Innendurchmesser, der größer ist als der Außendurchmesser der Zugstange 3 an ihrer umfänglichen Verdickung 31.

In Fig. 3 ist gezeigt, dass sich zur linearen Führung von Gabelelement 4 und Schaft 2 entlang dem Endabschnitt GE des Gabelelements 4 in proximaler Richtung axial zwei Stege 42 erstrecken. Korrespondieren dazu erstrecken sich entlang dem Endabschnitt SE des Schafts 2 von dessen Ende in Richtung der Schulter 21 zwei Ausnehmungen 23. Stege 42 und Ausnehmungen 23 bilden Führungsschienenelemente des Schafts 2 und des Gabelelements 4 und können für eine Montage schienenartig ineinandergeschoben werden. Die Stege 42 an dem Gabelelement 4 und die Ausnehmungen 23 an dem Schaft 2 sind zu den Ausnehmungen 22 des Schafts 2 und den Federplättchen 5 um 90 ° versetzt angeordnet, so dass die Federplättchen 5, wenn sie in die Hinterschnitte 43 bei Montage des chirurgischen Instruments 1 eingreifen, stabil von zwei Seiten gehalten werden, und keine Verdrehung der Bauteile zueinander auftreten kann.

Um den Endabschnitt GE des Gabelelements 4 und um den Endabschnitt SE des Schafts 2 ist ein Überwurfrohr 6 angeordnet. Das Überwurfrohr 6 weist zwei geschlossene Ausnehmungen 61 auf, die über den geschlossenen Ausnehmungen 22 des Endabschnitts SE des Schafts 2 liegen, wenn alle Bauteile in einer Montageanordnung vorliegen, wie Fig. 2 sie zeigt. Die geschlossenen Ausnehmungen 22 des Überwurfrohrs 6 überlappen sich nur in einem Teilbereich mit denen des Endabschnitts SE des Schafts 2 und enden vor der Auflagefläche 24 für die proximalen Enden der Federplättchen 5.

Die vorliegende Erfindung stellt ein chirurgisches Instrument 1 und ein Verfahren zu dessen Zerlegung bereit. Das chirurgische Instrument 1 hat einen Schaft 2, an dessen distalem Ende ein Werkzeug 101 lösbar gehalten ist, und eine in dem Schaft 2 angeordnete und in diesem längsbewegbare Zugstange 3 zum Bewegen des Werkzeugs 101. Dabei ist die Zugstange 3 von einem Gabelelement 4 distalseitig und von dem Schaft 2 proximalseitig umgeben. Das Gabelelement 4 weist an seiner dem Schaft 2 zugewandten Seite einen Endabschnitt GE mit verringertem Durchmesser auf, wobei benachbart zu einem Ende des Endabschnitts GE zumindest ein Hinterschnitt 43 vorliegt. Ferner hat der Schaft 2 an seiner dem Gabelelement 4 zugewandten Seite eine erste Schulter 21, von der aus sich ein Endabschnitt SE mit verringertem Durchmesser erstreckt. Entlang des Endabschnitts SE erstreckt sich zumindest eine Ausnehmung 22, und ein Innendurchmesser des Schafts 2 ist entlang eines distalen Teils des Endabschnitts SE aufgeweitet. In die Ausnehmung 22 des Schafts 2 ist ein längliches Federplättchen 5 eingelegt, das an seinem zu dem Gabelelement 4 weisenden Ende eine radial nach innen weisende Rastnase 51 aufweist, die jeweils mit dem Hinterschnitt 43 des Gabelelements 4 in Eingriff steht. Hierbei weist die Zugstange 3 an ihrem vor dem proximalen Ende des Gabelelements 4 liegenden Abschnitt eine umfängliche Verdickung 31 des Durchmessers auf. Das Federplättchen 5 hat einen Quersteg 52, der zu der Zugstange 3 weist und zwischen dem Ende des Gabelelements 4 und der umfänglichen Verdickung 31 der Zugstange 3 liegt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### BEZUGSZEICHENLISTE

- 1: Chirurgisches Instrument
- 2: Schaft
- 21: Schulter
- 22: Ausnehmung
- 23: Schulter
- 24: Auflagefläche
- 3: Zugstange
- 31: umfängliche Verdickung
- 4: Gabelelement
- 41: Schulter
- 42: Steg
- 43: Hinterschnitt
- 44: Ende
- 45: innere Bohrung
- 5: Federplättchen
- 51: Rastnase
- 52: Quersteg
- 53: proximales Ende
- 6: Überwurfrohr
- 61: Ausnehmung
- 10: Griff
- 101: Werkzeug

- GE: Endabschnitt Gabelelement
- SE: Endabschnitt Schaft

## Patentansprüche

1. Chirurgisches Instrument (1) mit einem Schaft (2), an dessen distalem Ende ein Werkzeug (101) lösbar gehalten ist, und mit einer in dem
Schaft (2) angeordneten und in diesem längsbewegbaren Zugstange (3) zum Bewegen des Werkzeugs (101),
wobei die Zugstange (3) von einem Gabelelement (4) distalseitig umgeben ist, und von dem Schaft (2) proximalseitig umgeben ist,
wobei
- das Gabelelement (4) an seiner dem Schaft (2) zugewandten Seite einen Endabschnitt (GE) aufweist, zu dessen Ende (44) benachbart zumindest ein Hinterschnitt (43) vorliegt,
- der Schaft (2) an seiner dem Gabelelement (4) zugewandten Seite eine erste Schulter (21) aufweist, von der aus sich ein Endabschnitt (SE) mit verringertem Durchmesser erstreckt, wobei entlang dem Endabschnitt (SE) sich zumindest eine Ausnehmung (22) erstreckt, und
wobei ein Innendurchmesser des Schafts (2) entlang einem distalen Teil des Endabschnitts (SE) aufgeweitet ist, und
wobei in die zumindest eine Ausnehmung (22) des Schafts (2) ein längliches Federplättchen (5) eingelegt ist, das an seinem zu dem Gabelelement (4) weisenden Ende eine radial nach innen weisende Rastnase (51) aufweist, die mit dem zumindest einen Hinterschnitt (43) des Gabelelements (4) in Eingriff steht, und wobei die Zugstange (3) an ihrem vor dem proximalen Ende des Gabelelements (4) liegenden Abschnitt eine umfängliche Verdickung (31) des Durchmessers aufweist, und wobei das Federplättchen (5) einen Quersteg (52) hat, der zu der Zugstange (3) weist und zwischen dem Ende des Gabelelements (4) und der umfänglichen Verdickung (31) der Zugstange (3) liegt, und
wobei die Verdickung (31) der Zugstange (3) auf den Quersteg (52) des Federplättchens (5) wirkt, sodass diese die Rastnase (51) radial nach außen drückt.

2. Chirurgisches Instrument (1) nach Anspruch 1,
**dadurch gekennzeichnet, dass**
- entlang dem Endabschnitt (GE) des Gabelelement (4) sich zwei Führungsschienenelemente erstrecken,
- entlang dem Endabschnitt (SE) des Schafts (2) sich zwei Führungsschienenelemente von dessen Ende in Richtung der Schulter (21) erstrecken,
wobei die zwei Führungsschienenelemente des Schafts (2) jeweils mit den Führungsschienenelementen des Gabelelements (4) verschiebbar zusammenwirken.

3. Chirurgisches Instrument (1) nach Anspruch 2,
**dadurch gekennzeichnet, dass**
die Führungsschienenelemente des Schafts (2) Ausnehmungen (23) oder Stege sind, und dass korrespondierend für das Zusammenwirken die Führungsschienenelementen des Gabelelements (4) Stege (42) oder Ausnehmungen oder Rillen sind.

4. Chirurgisches Instrument (1) nach Anspruch 2 oder 3,
**dadurch gekennzeichnet, dass**
die Führungsschienenelemente des Schafts (2) zu der zumindest einen Ausnehmung (22) des Schafts (2) um 90 ° versetzt angeordnet sind.

5. Chirurgisches Instrument (1) nach einem der vorstehenden Ansprüche,
**dadurch gekennzeichnet, dass**
im Montagezustand ein Überwurfrohr (6) um den Endabschnitt (GE) des Gabelelements (4) und um den Endabschnitt (SE) des Schafts (2) angeordnet ist.

6. Verfahren zur Zerlegung eines Chirurgisches Instruments (1) nach einem der Ansprüche 1 bis 5,
umfassend die Schritte
- in axialer Richtung am proximalen Ende der Zugstange (2) Druck ausüben auf die Zugstange (2), dabei bewegen der Zugstange (2) in distaler Richtung,
- dadurch bewegen der umfänglichen Verdickung (31) der Zugstange (2) gegen den Quersteg (52) des Federplättchens (5) bis in eine Überoffenposition der Zugstange (2), dabei
- radial nach außen bewegen der Rastnase (51) aus dem Hinterschnitt (43) des Gabelelements (4), und
- durch das Lösen der Verbindung von Gabelelement (4) und Schaft (2) lösen des Werkzeugs (101).

## Claims

1. A surgical instrument (1) with a shaft (2), at the distal end of which a tool (101) is detachably held, and with a tension rod (3) which is arranged in the shaft (2) and is longitudinally movable therein for moving the tool (101),
wherein the tension rod (3) is surrounded distally by a fork element (4),
and is surrounded proximally by the shaft (2),
wherein
- the fork element (4) has, on its side facing the shaft (2), an end section (GE), adjacent to the end (44) of which there is at least one undercut (43),
- the shaft (2) has, on its side facing the fork element (4), a first shoulder (21) from which an end section (SE) with reduced diameter extends, wherein at least one recess (22) extends along the end section (SE), and
wherein an inner diameter of the shaft (2) is widened along a distal part of the end section (SE), and
wherein into the at least one recess (22) of the shaft (2) is inserted an elongate spring plate (5), which, at its end pointing towards the fork element (4), has a radially inwardly pointing latching lug (51), which engages with the at least one undercut (43) of the fork element (4), and wherein the tension rod (3) has a circumferential thickened portion (31) of the diameter at its section lying in front of the proximal end of the fork element (4), and wherein the spring plate (5) has a transverse web (52) which points towards the tension rod (3) and lies between the end of the fork element (4) and the circumferential thickened portion (31) of the tension rod (3), and
wherein the thickened portion (31) of the tension rod (3) acts on the transverse web (52) of the spring plate (5) such that it presses the latching lug (51) radially outwards.

2. The surgical instrument (1) according to claim 1,
**characterised in that**
- two guide rail elements extend along the end section (GE) of the fork element (4),
- two guide rail elements extend along the end section (SE) of the shaft (2) from its end in the direction of the shoulder (21),
wherein the two guide rail elements of the shaft (2) each interact displaceably with the guide rail elements of the fork element (4).

3. The surgical instrument (1) according to claim 2,
**characterised in that**
the guide rail elements of the shaft (2) are recesses (23) or webs, and **in that**, correspondingly for the interaction, the guide rail elements of the fork element (4) are webs (42) or recesses or grooves.

4. The surgical instrument (1) according to claim 2 or 3,
**characterised in that**
the guide rail elements of the shaft (2) are arranged offset by 90° relative to the at least one recess (22) of the shaft (2).

5. The surgical instrument (1) according to one of the preceding claims,
**characterised in that**
in the assembled state, a retaining sleeve (6) is arranged around the end section (GE) of the fork element (4) and around the end section (SE) of the shaft (2).

6. A method for disassembling a surgical instrument (1) according to one of claims 1 to 5, comprising the steps
- exerting pressure on the tension rod (2) in the axial direction at the proximal end of the tension rod (2), thereby moving the tension rod (2) in the distal direction,
- thus moving the circumferential thickened portion (31) of the tension rod (2) against the transverse web (52) of the spring plate (5) into an over-open position of the tension rod (2), thereby
- moving the latching lug (51) radially outwards out of the undercut (43) of the fork element (4), and
- by loosening the connection between the fork element (4) and the shaft (2), releasing the tool (101).

## Revendications

1. Instrument chirurgical (1) comportant un arbre (2) à l'extrémité distale duquel un outil (101) est maintenu de manière amovible, et comportant une tige de traction (3) agencée dans l'arbre (2) et pouvant se déplacer longitudinalement dans celui-ci pour déplacer l'outil (101),
dans lequel la tige de traction (3) est entourée du côté distal par un élément fourche (4), et est entourée par l'arbre (2) du côté proximal,
dans lequel
- l'élément fourche (4) présente, sur son côté tourné vers l'arbre (2), une section d'extrémité (GE), au voisinage de l'extrémité (44) de laquelle se trouve au moins une contre-dépouille (43),
- l'arbre (2) présente, sur son côté tourné vers l'élément fourche (4), un premier épaulement (21) à partir duquel s'étend une section d'extrémité (SE) de diamètre réduit, dans lequel au moins un évidement (22) s'étend le long de la section d'extrémité (SE), et dans lequel un diamètre interne de l'arbre (2) est élargi le long d'une partie distale de la section d'extrémité (SE), et
dans lequel une plaquette de ressort (5) allongée est insérée dans l'au moins un évidement (22) de l'arbre (2), laquelle présente à son extrémité orientée vers l'élément fourche (4) un ergot d'arrêt (51) orienté radialement vers l'intérieur, qui est en prise avec l'au moins une contre-dépouille (43) de l'élément fourche (4), et dans lequel la tige de traction (3) présente un épaississement (31) circonférentiel de diamètre au niveau de sa section située en avant de l'extrémité proximale de l'élément fourche (4), et dans lequel la plaquette de ressort (5) a une nervure transversale (52) qui fait face à la tige de traction (3) et qui est située entre l'extrémité de l'élément fourche (4) et l'épaississement (31) circonférentiel de la tige de traction (3), et
dans lequel l'épaississement (31) de la barre de traction (3) agit sur la nervure transversale (52) de la plaquette de ressort (5), de sorte que celle-ci pousse l'ergot d'arrêt (51) radialement vers l'extérieur.

2. Instrument chirurgical (1) selon la revendication 1,
**caractérisé en ce que**
- deux éléments de rail de guidage s'étendent le long de la section d'extrémité (GE) de l'élément fourche (4),
- le long de la section d'extrémité (SE) de l'arbre (2), deux éléments de rail de guidage s'étendent depuis l'extrémité de celui-ci en direction de l'épaulement (21),
dans lequel les deux éléments de rail de guidage de l'arbre (2) coopèrent de manière coulissante respectivement avec les éléments de rail de guidage de l'élément fourche (4).

3. Instrument chirurgical (1) selon la revendication 2
**caractérisé en ce que**
les éléments de rail de guidage de l'arbre (2) sont des évidements (23) ou des nervures, et **en ce que**, de manière correspondante pour la coopération, les éléments de rail de guidage de l'élément fourche (4) sont des nervures (42) ou des évidements ou des rainures.

4. Instrument chirurgical (1) selon la revendication 2 ou 3,
**caractérisé en ce que**
les éléments de rail de guidage de l'arbre (2) sont agencés de manière décalée de 90 ° par rapport à l'au moins un évidement (22) de l'arbre (2).

5. Instrument chirurgical (1) selon l'une des revendications précédentes,
**caractérisé en ce que**
à l'état monté, un tube de raccordement (6) est agencé autour de la section d'extrémité (GE) de l'élément fourche (4) et autour de la section d'extrémité (SE) de l'arbre (2).

6. Procédé de démontage d'un instrument chirurgical (1) selon l'une des
revendications 1 à 5,
comprend les étapes
- d'exercice d'une pression sur la tige de traction (2) dans la direction axiale au niveau de l'extrémité proximale de la tige de traction (2), tout en déplaçant la tige de traction (2) dans la direction distale,
- de déplacement ainsi de l'épaississement (31) circonférentiel de la tige de traction (2) contre la nervure transversale (52) de la plaque de ressort (5) jusqu'à une position de surouverture de la tige de traction (2), ainsi
- de déplacement radial vers l'extérieur de l'ergot d'arrêt (51) hors de la contre-dépouille (43) de l'élément fourche (4), et
- par desserrage de la liaison entre l'élément fourche (4) et l'arbre (2) desserrage de l'outil (101).
